# EUROPEAN PATENT APPLICATION

(11) **EP 1 700 863 A1**
(43) Date of publication of application: **13.09.2006**
(21) Application number: 05075540.4
(22) Date of filing: 04.03.2005
(51) Int. Cl.: C07K 7/66, A61K 38/04

(54) **Means and methods for producing polymers comprising peptide in a side chain**

(71) Applicant: Stichting voor de Technische Wetenschappen, 3527 JP Utrecht (NL)
(72) Inventor: Ayres, Lee, 6521 DA Nijmwgen (NL); Grotenbreg, Gijsbert Marnix, Sommerville, MA 02143 (US); Overhand, Mark, 2312 SZ Leiden (NL); van Hest, Jan Cornelis Maria, 6522 DL Nijmegen (NL)
(74) Representative: Winckels, Johannes Hubertus F.

(57) **Abstract**

The invention provides means and methods for producing polymers via mediated radical polymerisation wherein monomers comprising peptides in a side chain are polymerised. One of the applications of such polymers is the coating of surfaces with antibacterial (cyclic) peptides.

## Description

The invention relates to the field of polymer-peptide chimera and uses thereof. The invention in particular relates to chimera produced via mediated or living radical polymerisation.

Complexes containing both proteins and polymers are typically produced by covalently linking the two after both are generated. In the present invention such complexes are produced by linking monomers that have (poly)peptides in their side chains. Linking of monomers has the advantage of strict control over the number of (poly)peptides that are present in the polymer. Limiting factors such as diffusion of proteinaceous components into polymer structure to find the linkage sites, distribution of the polymer chains and cross-reactivity of the reactive (leaving) groups to link the (poly)peptide chains are not a problem with a method of the invention. An additional level of control over the structure of the produced complex is in the present invention obtained by polymerising in so-called 'mediated radical' or 'living chain' polymerisation reactions. These reactions are characterised by polymer chains that grow linearly with time (first order kinetics). This is typically achieved by providing a capping agent in the reaction that can associate with the growing polymer chain. When associated, the capping agent binds the free radical thereby effectively preventing chain elongation. Binding of the capping agent to the polymer is reversible and release of the capping agent produces a free radical on the polymer chain and thereby provides it with an initiation site for polymerisation. By introducing (poly)peptide in the side chains of the monomers, the present invention generates complexes that share at least some of the properties of polymers and of (poly)peptides.

In the present invention it was found that polymerisation of monomers comprising a (poly)peptide (or functional equivalents thereof) comprising at least 7 amino acids (of functional equivalents thereof) in peptidic linkage in a side chain could be efficiently polymerised using living chain or mediated polymerisation.
This type of polymerisation is among others dependent on the equilibrium between free versus capped polymer in the growing polymer chain. The control over the polymerisation process is based on two principles. Initiation should be fast to allow for a constant concentration of growing polymer and the majority of the species should be dormant (capped) to reduce inadvertent termination. On the other hand, if the equilibrium is too much in favour of the capped, the chain will grow only slowly. In the present invention it was found that efficient polymerisation of monomers comprising at least 7 amino acids was achieved when the reaction temperature was higher than 50°C. Inadvertent chain termination is limited under these conditions, while growth of the polymer chain follows first order kinetics. Thus the invention in one aspect provides a method for producing a polymer comprising polymerising at least two monomers to an initiator in the presence of a capping agent that is reversibly associated with the growing polymer chain, wherein at least one of said monomers comprises a (poly)peptide or a functional equivalent thereof comprising at least 7 amino acids or functional equivalents thereof. In a preferred embodiment said polymerisation step is performed in the presence of a metal complex that reversibly associates with said capping agent thereby providing said method with a switching mechanism that switches said polymer between a not growing capped (dormant) state and an uncapped growing (living) state.

By having at least 7 amino acids in the side chain it is possible to produce complexes wherein the (poly)peptide itself exhibits a function. With less than 7 it is often not possible to exhibit a function without the aid of neighbouring side chains. Moreover, as will be described in more detail below, having at least 7 amino acids in a side chain requires different polymerisation conditions when compared to conditions that are suited for, for example, polymerisation of monomers having 4 or 5 amino acids in side chains. This is probably due to the relative bulkiness of the side chain. Said modified conditions are suitable for the polymerisation of monomers comprising peptides comprising at least 7 and preferably at least 10 amino acids or functional equivalents thereof in peptidic linkage. In a preferred embodiment said side chain comprises less than 100 amino acids. Preferably said side chain comprises less than 80 amino acids and more preferably less than 20 amino acids.

A polymer is in principle a molecule composed of repeated subunits. This definition would include polymeric substances such as polypeptides, nucleic acids and the like. This is not the intended definition in the present invention. Herein, a polymer is a molecule having repeated subunits that are normally linked to each other via radical polymerisation. This definition excludes biopolymers since these are normally produced in a different way. This definition does not exclude polymers that are arrived at using a different method but that are normally produced by means of radical polymerisation. The polymer may be modified during or after polymerisation. The term "polymerising" in the present invention thus refers to radical polymerisation. Prior to incorporation into the polymer chain, the subunits are referred to as monomers. Monomers are compounds capable of being incorporated into a propagating polymer chain thereby consuming a radical on the recipient initiator or polymer chain (at one end of the monomer) and presenting a new radical on the other end of the now incorporated monomer. A monomer can itself be a polymeric or oligomeric compound. An initiator is typically a compound that provides the first radical(s) for linking monomers in the polymerisation. During polymerisation, the growing polymer can be seen as a sort of initiator for incorporation of a subsequent monomer.

Polymerisation is conducted in the presence of a capping agent (mediator). The process involves the presence of a capping agent (nitroxyl radical, halogen atom, organometal moiety, thioester functionality or other molecular fragment) to react reversibly with the propagating radical to generate a non-radical species which is unreactive. In this bound state the propagating radical is stable or dormant and unable to participate in termination or chain transfer processes. Chain extension is controlled by the position of equilibrium between the dormant and active or free propagating radical. Under appropriate conditions very good mediation of the radical polymerisation may be achieved and some of the characteristics of living polymerisation may be observed, e.g., ready chain extension to form block polymers, narrow polydispersity, molecular weight control (reflecting the monomer to initiator molar ratio for systems in which preformed initiators/mediators are used), end-group purity, etc.

In a preferred embodiment polymerisation is performed using nitroxyl mediated radical polymerisation (NMRP), reversible addition fragmentation transfer polymerization (RAFT) or atom transfer radical polymerisation (ATRP). All three processes incorporate characteristics of mediated and/or living polymerisation reactions which vary with the exact nature of the polymerisation and the reaction conditions. These processes have been widely explored and their scope and limitations are largely established. Thus the terms NMRP, RAFT and ATRP are functionally descriptive, i.e. it is well understood to what they refer. In these processes, unwanted side-reactions are suppressed (to a greater or lesser degree, depending on the reaction conditions) and the resulting polymer is more nearly ideal than that which can be obtained in the absence of the capping agent. The ATRP process is particularly preferred in the present invention. For a recent review of ATRP and a non-limiting list of polymers/monomers/initiators and capping agents that can be generated and or used in ATRP, reference is made to Coessens³⁰, V. et al (2001)The monomers listed in the mentioned references are suitable backbones to which amino acids and/or peptides may be linked in the side chain as desired. In a preferred embodiment the monomer backbone comprises a methacrylate.

In ATRP, capping and decapping of the growing polymer is typically catalysed by a metal complex. The catalyst determines the equilibrium between the dormant (capped) and growing (uncapped) polymer chain. This equilibrium is important because it determines parameters such as the rate of polymer growth and inadvertent chain termination. In the present invention it has been found that catalysts of the Cu(I)/Cu(II) type are suited for polymerising monomers comprising at least 7 amino acids or functional equivalents thereof in peptidic linkage. Other catalysts that can be used are based on transition metal complexes, especially complexes comprising Fe, Pd, Ni, Rh or Ru, or any other catalyst known to be active in the Kharasch addition, more particular FeCl₂ /FeCl₃ combined with ligands containing amine, phosphorus or a combination, more preferably organic acid ligands such as isophthalic acid and iminodiacetic acid, Pd(OAc)₂/PPh₃/CCl₄, arylNi^{II} complexes, more specifically NiBr₂(PPh₃)₂ and Ni{o,o'(CH₂NMe₂)₂C₆H₃}Br, Rh complexes known as the Wilkinson catalyst and Ru phosphorus complexes in combination with a Lewis acid, or more preferably [RuCl(Cp)(PPh₃)₂], [RuCl(Ind)(PPh₃)₂] and [RuCl(Cp*)(PPh₃)₂]. Metal complexes comprising copper are preferred. Preferably the copper ion is complexed with ligands known to be active in the Kharasch addition, in particular containing at least 2 nitrogens spaced by 2 or 3 C atoms, more preferably 2,2' bipyridine and derivatives thereof, salen complexes and derivatives thereof, multiamine ligands, such as tetramethyl ethylene diamine (TMEDA), pentamethyl diethylene triamine (PMDETA), hexamethyl triethylene tetraamine (HMTETA) and derivatives thereof, TREN and Me₆TREN, cyclam and N-alkyl-2-pyridylmethanimines. In a particularly preferred embodiment said metal complex comprises Cu(I) or Cu(II) complexed with a multi-amine ligand, preferably with HMTETA or PMDETA.

The (poly)peptide in the side chain of a monomer can be any type of peptide. The side chain or the peptide therein can contain one or more additional polymerisation initiation sites. A monomer can function as a chain propagator and both as a chain propagator and a chain initiator. The latter is, for instance, used for the production of branched molecules. Polymerisation is preferably achieved using a single type of monomer. In another embodiment, more than one type of monomer is added to the polymerisation reaction. This is often used for the production of co-polymers. A peptide in the side chain is preferable a cyclopeptide.

Cyclopeptides can be synthesized both biologically or synthetically^{40,41}. There are several ways in which (poly)peptide based monomers can be produced⁴²⁻⁴⁴. Preferred methods for producing peptide-based monomers are synthesis of the structure from the C to the N terminus to prevent epimerization, synthesis on solid support to allow cleaning up procedures, and in which care should be taken in the deprotection step concerning the chemical stability of the polymerisation handle. Polymers of the invention preferably comprise linear (poly)peptides that can fold into a helix or beta-sheet and cyclic (poly)peptides. A polypeptide having a β-sheet or a β-helical turn is more resistant to denaturation. A first reason is that a β-sheet usually forms a tight structure that resists denaturation more effectively than other secondary and tertiary (poly)peptide structures. A second reason is that a β-sheet or a β-helical turn more easily refolds into the original conformation upon removal of the cause of denaturation. Such denaturation conditions may for instance occur during or subsequent to the polymerisation of the monomers. Cyclic (poly)peptides are also more resistant to denaturation.

A functional equivalent of a peptide is for instance a peptidomimetic. A peptidomimetic is a compound that mimics or blocks an effect of a peptide or a protein motif. In a biological system, the peptidomimetic mimics or blocks biological effects of a peptide or a protein motif. In such case the peptidomimetic has the potential to act as a drug molecule. No matter what is the nature of its chemical structure this is a synthetic compound. In biological systems a peptidomimetic aims at being an active agent to achieve a similar effect in kind, not necessarily in amount, as the peptide it mimics. Non-limiting methods for generating peptidomimetics and peptidomimetics themselves are disclosed in Patch and Barron (2002)⁴⁵ and Matsoukas *et al* (2001)⁴⁶. A further equivalent of a peptide is a peptide or mimetic that has been biologically or chemically modified during or after synthesis. Such modification may include lipidation, meristylation and/or glycosylation etc. A functional equivalent of an amino acid has the same capacity to form an integral part of a (poly)peptide chain as an amino acid in kind not necessarily in amount. Many different amino acid analogues have been generated. They vary widely including simple modification (hydroxylation) to complete redesigns such as so-called β-amino acids (having a modified backbone) to artificial amino acids that have artificial side chains having no counterpart in nature. Non-limiting examples of β-amino acids are described in "Enantioselective synthesis of beta-amino acids" Ed. E. Juaristi, Wiley-VCH 1997 , non-limiting examples of artificial amino acids are described in Adv. Mat. 1997, 9, 299⁴⁷ **Rutjes FPJT** et **al (2000)**^{**48**} , Wang L (2005)⁴⁹, Ma, James S (2003)⁵⁰.

The invention further provides a polymer comprising at least one peptide or a functional equivalent thereof comprising at least 7 amino acids or functional equivalents thereof in a side chain. The polymer is preferably obtained by a method of the invention. In a particularly preferred embodiment said peptide is capable of inhibiting the growth of bacteria. Preferably it comprises bacteriostatic activity and preferably bactericidal activity.

The invention further provides a monomer that is polymerisable through radical polymerisation wherein said radical polymerisable monomer comprises at least 7 amino acids and preferably at least 10 amino acids in peptidic linkage in a side chain. In a preferred embodiment said monomer comprises less than 100 amino acids in peptidic linkage. In a particularly preferred embodiment said monomer comprises less than 20 amino acids in peptidic linkage. A monomer that is polymerisable through radical polymerisation comprises at least one reactive carbon-carbon double bond.

Antimicrobial materials have become increasingly important in many aspects of our daily lives. Food hygiene, protection against bacterial infections in the biomedical field and prolonged preservation of produce are just three areas of application for which there is a demand for improved antibiotic materials. In particular much research has been conducted to prepare antimicrobial coatings. Non-covalent incorporation of antibiotic agents has been studied most extensively, but has some drawbacks such as limited long-term effectiveness, possibility of resistance development and low efficiency. These problems can partly be circumvented through the use of covalently immobilized antibiotics. In this case however care has to be taken not to destroy the antibiotic activity via the chemical coupling process.

Antibacterial peptides that disrupt membrane functioning, are of interest in the present invention because bacteria cannot develop resistance against these structures. The development of peptide mimetics that are built-up out of other than L-α-amino acids has furthermore allowed the introduction of resistance against biodegradation. This makes antibiotic peptides, based on cyclopeptides and β-peptides very interesting candidates for safe and prolonged usage. The incorporation of these peptides into coatings via covalent approaches has however not been investigated until now.

In the present invention a new class of antibiotic compounds has been generated by synthesizing tailor made polymers comprising antibiotic cyclic and β-peptides. The advantages of these peptides are combined with the advantages of constructing polymeric antibiotics via controlled radical polymerisation methods, such as ease of immobilization and the ability to fine tune the antibiotic properties, by varying the density and number of cyclic and/or β-peptide compounds along the polymer backbone. The new class of antibiotics has long term activity and is easy to apply to different types of surfaces and is therefore suited for a wide variety of applications.

In the present invention a generic strategy is used for the construction of antibacterial coatings which are easy to use, do not cause resistance in the sensitive bacteria and do not easily degrade upon use. This is achieved through usage of peptide-mimetic compounds that function as stable antibiotics, without the risk of resistance development and through the application of a controlled polymerisation technique for anchoring of the active compounds into polymers. Polymers can be generated that allow efficient immobilization on a wide variety of surfaces.

Much research has been conducted over the years to develop antimicrobial coatings, for example in active food packaging¹. An efficient protective layer can be generated by introducing the antimicrobial ingredient into the coating in an active form. Physical adsorption of antimicrobial compounds onto a surface or incorporation into a coating layer are probably the most straightforward methods, however with some serious drawbacks. The active ingredient is not covalently bound and therefore is slowly released into the environment. Especially in the food packaging industry slow release of antibacterial agents is an undesirable property. Because this release is quite often not efficient, much of the active compound remains trapped within the coating and the antibiotic surface activity disappears rather quickly. Furthermore, this method can cause the danger that bacteria are exposed to low levels of antibiotic agents, which enables them to develop resistance. Especially in the biomedical field the limitation of long-term effectiveness has been regarded as a major problem².

Only a few examples are known in which the antibacterial agent is covalently coupled to the surface³⁻⁵. Although longer lasting activity can be obtained, this approach/method is more complicated, because it is necessary for covalent attachment that both the surface and the antibacterial agent contain functional moieties that can react with each other. This chemical coupling of the agent can result in a diminished activity, because of the chemical modification and because the agent is not able any more to freely interact with the bacteria. One solution to this problem is the use of spacers between attachment point and antibacterial moiety.

Many different types of antibacterial agents have been studied over the years. Additives to coatings include quaternary ammonium salts, horse radish (allyl isothiocyanate), silver and enzymes such as lysozyme and chitinase¹. Especially silver has found widespread application in the biomedical field, although the specific in vivo activity is still under dispute^{2,6}. The activity and stability of enzymes is normally too low for many applications.

A preferred class of bacteriostatic or bacteriocidal (poly)peptides that is incorporated into monomers of the invention is the family of naturally occurring membrane active peptide toxins and antibiotics, such as magainins, bombolitin, mellitin and gramicidins⁷ and functional analogues thereof. Functional analogues of these bacteriocidal compounds comprise the same bacteriocidal activity in kind not necessarily in amount.

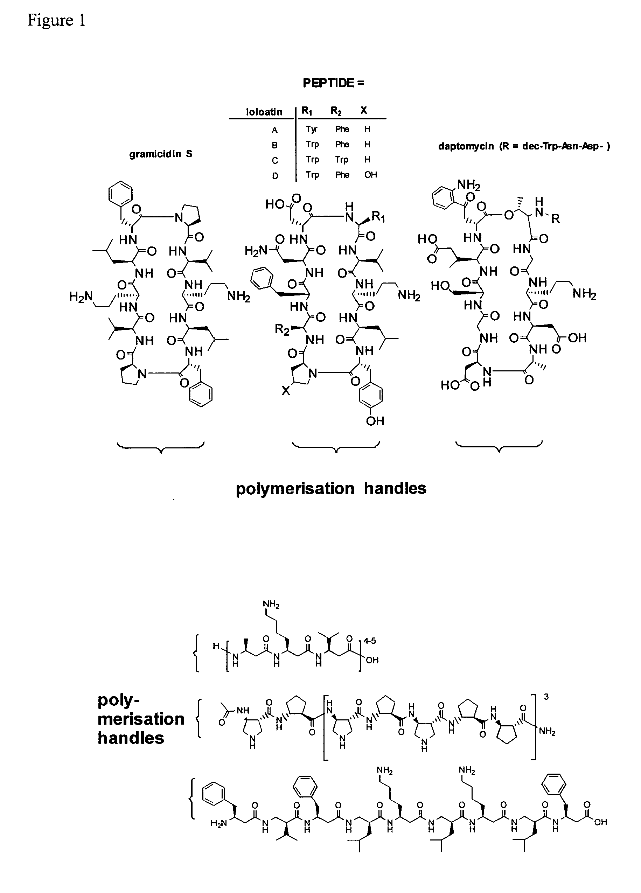

In a particularly preferred embodiment a monomer comprises Gramicidin S, loloatin and/or daptomycin according to Figure 1, or a functional derivative thereof, preferably as depicted in Figure 1. These peptides have been around for millions of years without bacteria developing resistance against them. Common features of these peptides are their amphiphatic structure, hydrophilicity and the abundance of positively charged amino acid residues. This allows the peptides a strong interaction with the negatively charged phospholipids in the cell membrane of bacteria. Because membranes of plants and animals are composed of lipids with no net charge, these peptides function specifically against bacteria. The precise working mechanism is not completely known, but most theories take into account that the interaction between peptide and phospholipids causes a detrimental structural change in the bacterial cell membrane that disrupts its functioning.

Because of the abovementioned characteristics, specific peptide sequences have been included in antibacterial coatings. Their application potential however has been until now rather limited, because it was unexpectedly observed that, although high *in vitro* activity was demonstrated, they were only effective in animal tests in doses that were close to the toxic dose. In order to prevent the toxicity problem it was proposed to covalently immobilize antibiotic peptides. The potential of this approach was investigated with e.g. magainin and synthetic analogues, which were covalently linked to polymeric beads via solid phase peptide synthesis, using the beads as peptide resin^{4,5}. Activity of the antibiotic peptides could be demonstrated *in vitro.* The difficulty with this approach is that all synthetic steps had to be performed on the polymer surface to build up the desired antibacterial peptide, and it could therefore not be excluded that by-products were formed, such as truncated peptides, which cannot be removed afterwards. Especially for biomedical applications, this method therefore has its limitations.

Another problem that arises from the use of natural peptides is that they are still susceptible to protease activity, which would lead to a decrease in antibacterial activity in biological environments. To circumvent this issue, peptides that consist of other than L-α-amino acids have been synthesized and tested. It was shown that peptides built up out of D-α amino acids displayed similar activity as their natural counterparts. More interesting, cyclic peptides constructed out of alternating D- and L-α amino acids and peptides that were entirely composed of β-amino acids were found to be very efficient as antibiotics⁸⁻¹². Especially the latter class of compounds shows excellent resistance against proteases. Covalent immobilization of these peptide mimetics however has not been explored thus far.

Quite recently, polymers with intrinsic antibacterial properties have been developed¹³⁻¹⁸. One of the advantages of using polymers instead of single compounds is that covalent incorporation of the active compound is taken care of efficiently, the spatial distribution and number of antibacterial compounds can be arranged when control over polymer length and composition can be established, leading to optimal usage of the active antibacterial compounds, and immobilization processes on polymer surfaces are easier to perform when the correct polymer-polymer interactions are built in the design.

Until now the potential of constructing polymeric antibiotics has been largely unexplored. Examples have been reported in which the polymer upon degradation released antibiotic compounds¹³. Degrado et al. constructed a polyamide that could mimic the structure of magainin, and therefore also exhibited antibiotic activity¹⁴. Most of the research on antibiotic polymers has been conducted on positively charged polymers, based on the notion that cationic charge is important for antibiotic activity and on the fact that quaternary ammonium salts function well as antibiotic agents. Recently, Klibanov et al. have shown that by applying a polymer containing ammonium groups which are connected via a spacer to the backbone, efficient antibiotic surfaces could be obtained¹⁵⁻¹⁸. These surfaces are not only active in aqueous environment, but function also very efficiently in air, and are therefore suitable for protection against airborne bacteria. The positively charged surface even attracts bacteria from the environment. The bacteria are strongly adhered to the surface, which impedes cell division or even kills the bacteria, but a possible drawback is that a biomass film will be developed on the surface which, when thick enough, renders the polycationic polymer layer inactive, and could result in problems for in vivo applications. In one of their papers¹⁵ Klibanov et al. claimed that polycationic surfaces were almost equally active against Gram negative and Gram positive bacteria, though other studies showed that Gram positive bacteria are considerably less affected by cationic charges¹⁹.

One of the reasons for the rather limited use of polymers in antibacterial applications has been the difficulty of synthesizing peptide-functional polymers in a controlled fashion²⁰. In the present invention it is shown that new controlled radical polymerisation methods, in particular Atom Transfer Radical Polymerisation, allow the introduction of a peptide in the side chain of a polymer. Different polymer architectures can be prepared, such as diblock and triblock copolymers. A variety of peptide fragments are incorporated, from silk and elastin-mimetic tetra- and pentapeptides to cyclic decapeptides such as Gramicidin S.

The gramicidins are a class of linear and cyclic amphiphilic oligopeptides that are able to disrupt membrane functioning. Several other similar membrane disrupting peptides are known, including magainins, bombolitin, mellitin, loloatins and daptomycin. Oligopeptides are readily assembled using solid phase approaches. Subsequent cyclization reactions may be performed in solution^{51,52}.

Using a method of the invention it is possible to vary the density and number of bacteriocidal or bacteriostatic peptides along the polymer backbone. This is also true for cyclic or β-peptide compounds. The new class of antibiotics has long term activity and is easy to apply to different types of surfaces and therefore suited for a wide variety of applications. Furthermore, because specific peptide sequences can be introduced, targeting of certain types of bacteria is achievable, in contrast to e.g. the generic polycationic materials. The new (poly)peptide-polymers of the invention can be applied as protective layers for tulip bulbs, textile coatings, antibiotic bone cement and antibiotic coatings for medical devices. Thus the invention further provides the use a polymer of the invention for interacting with biological material, or for coating a surface. The invention also provides the use of a polymer of the invention for at least inhibiting bacterial growth.

One of the preferred applications of a polymer of the invention is in biomedical devices that are in contact with an individual. Areas of contact of the body with the devices or parts thereof are very prone to bacterial infection. These surface areas are therefore preferably treated with a polymer of the invention. Treatment with a polymer of the invention will at least reduce the growth of bacteria when compared to untreated surface areas. Thus the invention further provides an apparatus comprising tubing coated with a polymer of the invention. The invention also provides a device comprising a surface for use at an interface between a bacteria free and a bacteria containing environment, said surface comprising a polymer of the invention. Preferably said device is a medical device. Preferably a disposable device. In yet another aspect the invention provides a coating comprising a polymer of the invention.

### Examples

### Peptide synthesis and development

With solid phase peptide synthesis four types of antibiotic peptide monomers are constructed for further evaluation: a) Gramicidin S, which is active against Gram positive bacteria, b) a second cyclic decapeptide, loloatin A-D, active against both Gram positive and Gram negative bacteria, c) the clinically approved daptomycin²² and d) helical β-peptides, consisting of 9-17 residues (Figure 1) The cyclic oligomers composed of "normal" α-amino acids and the linear β-peptides are stable against peptidases.

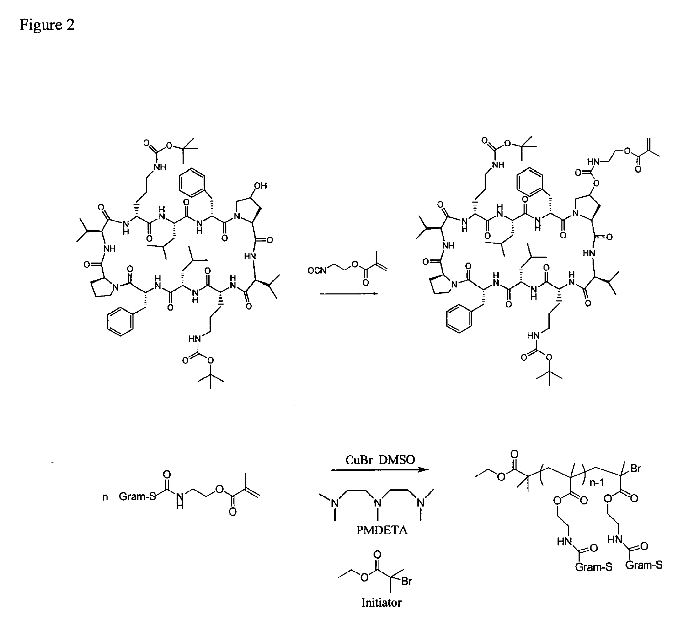

Controlled polymerisation via Atom Transfer Radical Polymerisation (ATRP) is most efficient when methacrylate moieties are introduced. The strategy for the preparation of well defined side chain peptide polymers is outlined in figure 2. One of the proline residues of the cyclic decapeptide gramicidin S was replaced during peptide synthesis by a hydroxyproline moiety. This modification does not affect the assembly behaviour and antibacterial character of the peptide. The introduction of the hydroxyl group allowed modification of the peptide with a methacrylate moiety via reaction with isocyanatoethyl methacrylate. This monomer was polymerised at 55°C under ATRP conditions, using CuBr/penta methyl diethylene triamine (PMDETA) as catalyst and DMSO as solvent. A degree of polymerisation of 20 was achieved. First order kinetics with respect to monomer consumption, as well as the very narrow molecular weight distribution (1.09) clearly indicated that control over polymerisation was obtained.

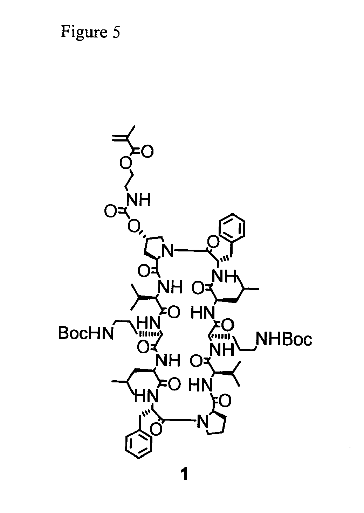

A typical experiment is the following: Polymerisation of a Gramicidin S based monomer, was performed using ATRP with the following conditions, 167 mg Gramicidin S monomer 1 (figure 5) (0.125 mmol), was dissolved in 0.5 ml DMSO-*d*₆ along with 1.27 mg CuCl (0.0125 mmol) and 0.75 µl penta methyl diethylene triamine (PMDETA) (0.0125 mmol). Finally 0.86 µl (0.00625 mmol) ethyl 2-bromoisobutyrate (EBIB) was added. This mixture was then heated to 55°C. After 15½ hours the conversion reached 65 % and the polymerisation was stopped. The polymer was then precipitated in a water/EDTA (2.5g per 100ml) solution.

This demonstrates that well-defined peptide polymers are easily accessible via the synthetic route developed.

We introduced specific polymerisation handles at all the other peptides as outlined in Figure 1. Because the handle of attachment to the polymer carrier material does not interfere with the bioactivity of the peptide^{23,24} care has to be taken to attach the methacrylate moiety to the end of the peptide or to a specifically introduced amino acid moiety which is not involved in the antimicrobial behaviour. In order to allow enough freedom for interaction with the bacteria, besides a direct coupling of the methacrylate moiety, spacer moieties can be introduced. In particular ethylene glycol based spacers of 1 to 10 repeats are preferred.

Several β-amino acid building blocks suitable for solid phase synthesis are commercially available, others will be synthesized following described procedures. Gramicidin S is not only active against bacteria, but also has a strong haemolytic effect, which could limit its application of a gramicidin polymer for bio-medical purposes On the other hand, the haemolytic activity of the beta-peptides as outlined in Figure 1 varies ²⁵⁻²⁷ , suggesting that fine-tuning of the molecular structures to reduce the toxic properties may be a viable approach. Therefore, after the first synthetic round of synthesizing the backbone unmodified peptides containing the polymerisable handles, backbone modifications are made via the introduction of amino acid mimetics, such as sugar amino acids²⁸⁻³⁴.

The introduction of amino acid analogues into gramicidin S has already been investigated successfully and has proven the feasibility of this approach. A series of modified antibiotic peptides will then become available for incorporation into polymers and for testing on antimicrobial behaviour. The feed back from the other parts of the program will result in an iteration that will lead to the optimal design of the antibiotic peptides against Gram positive and Gram negative bacteria.

### Peptide polymerisation

Immobilization of peptides via polymerisation is achieved in various ways. First, as a reference point, the traditional method for surface functionalisation via adsorption onto/blending in a polymer layer of the synthesized peptides is applied. Next the polymerisation characteristics of the different methacrylate functionalised peptides is applied. This is done in first instance via a free radical polymerisation process, which is photochemically initiated. Polymerisations are conducted of the pure peptides and of mixtures of the peptides with methyl methacrylate (MMA). After finding the optimal polymerisation conditions, coatings are made with varying amounts of peptides. The surfaces have antibacterial activity.

Control over the density of the antibacterial peptides and the number of moieties per polymer chain is achieved by using the controlled polymerisation technique of ATRP is used. This technique is well suited for the construction of well-defined polymers, even for antibiotic compounds such as gramicidin S. First homopolymers are made with varying degree of polymerisation. Via spin-coating, films are constructed that can be investigated on their antibacterial properties. In all cases care is taken to remove extensively the Cu catalyst from the polymer. To vary the density of peptide moieties, both random and block copolymer structures with MMA will be prepared.
Although all the antimicrobial peptides as depicted in figure 1 are known to interact with bacterial membranes, they are quite heterogeneous in their impact on these membranes^{27,35}. By synthesizing both well-defined rigid as well as flexible polymers it is possible to design antimicrobial efficiency as a function of the spatial orientation of the attached peptides. A further extension of the ATRP technology is to use functional initiators (Scheme 3) which allow surface grafting of the polymers, leading to the formation of surface brushes, with in case of initiator III a protective polyethylene glycol layer.

This polyethylene glycol layer is very useful to prevent attachment of cell debris to the surface, leading to both an active and passive form of protection.

### Characterization and Evaluation of the antibiotic activity

All peptides, peptide monomers and polymers are characterized with NMR spectroscopy, mass spectrometry and, where applicable, elemental analysis. The structural conformation is investigated with infrared spectroscopy, and possible differences between peptide and peptide polymers are analysed. The polymers are characterized by size exclusion chromatography, in order to determine the polydispersity, and with differential scanning calorimetry, to obtain knowledge about the physical properties of the polymers, such as glass transition temperature.
The novel materials are analysed with AAS to determine the residual metal content (copper ions will disturb bacterial growth), as well as gel filtration analysis to exclude the presence of added ligands.

Antibacterial behaviour is assessed at two different levels, i.e. effects on surface growth of initially adhering bacteria and antibacterial effects on bacterial growth in three dimensions. First, antibacterial activity with regard to inhibition of surface growth is determined in a parallel plate flow chamber. With this equipment, owing to the use of image analysis methods, distinctions are made between initial adhesion of bacteria, subsequent surface growth and cell detachment. These processes are directly visualized via phase contrast microscopy, in combination with a CCD camera and image analyser. Initial bacterial adhesion is investigated by using a suspension of micro-organisms in a buffer solution. For surface growth, the adhering micro-organisms are then allowed to grow in growth medium for a predetermined amount of time. Eradication is examined by introducing shear in the flow chamber by passing through air bubbles, or by applying a surfactant solution.

Three dimensional growth of adhering organisms is studied with a modified Robbins device (MRD), which is a hollow rectangular cubicle with 10 holes, in which plugs coated with antibacterial polymers are introduced. The coatings are first inoculated with bacteria in the MRD, followed by treatment for 3 days with a flow of growth medium. After this period, the plugs are removed from the MRD. Adhering biofilms will be stained using the BacLight system for observation of dead (red fluorescent) and green (living organisms) in a Confocal Scanning laser Microscope (CSLM). This device allows therefore for a fast and efficient screening of many different antibacterial coatings.

Clinical isolates from infected biomaterials from a large collection are used to test the produced polymers. The most successful coatings are therefore tested on many different types of bacteria, and the generic antibacterial characteristics are investigated.

### Brief description of the drawings

Figure 1: Molecular structure of the cationic α-peptides gramicidin, loloatin A-D, daptomycin and three structurally related cationic β-peptides antibiotics.
Figure 2: Preparation of well defined peptide based polymers by ATRP; a) functionalisation of gramicidin S with methacrylate handle; b) ATRP of gramicidin S monomer

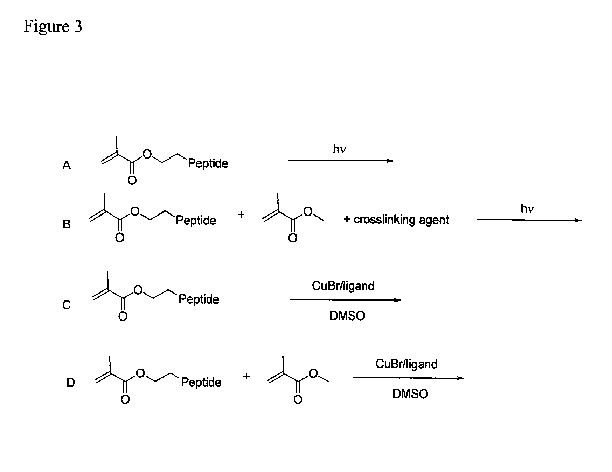

Figure 3: Different approaches to incorporate antibacterial peptides into polymers: A: Free radical photochemical polymerisation; B: Free radical copolymerisation; C: Atom Transfer Radical polymerisation (ATRP) for the construction of well-defined polymers; D: copolymerisation with ATRP.

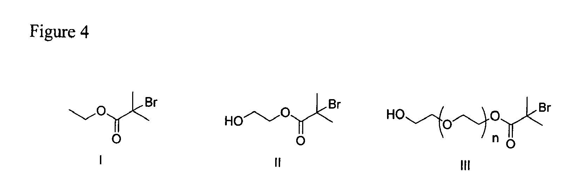

Figure 4: **I:** standard ATRP initiator, **II:** hydroxyl-functional initiator, useful for direct grafting on polymer surfaces, **III** poly(ethylene glycol) modified functional initiator
Figure 5. Gramicidin S based monomer 1

### References

1. Appendini, P.; Hotchkiss, J.H. *Innovative Food Science & Emerging Technologies.* **2002,** *3*, 113-126.
2. Schierholz, J.M.; Yucel, N.; Rump, A.F.E.; Beuth, J.; Pulverer, G. *International Journal of Antimicrobial Agents* **2002***, 19,* 511-516*.*
3. Laporte, D.C.; Rosenthal, K.S.; Storm, D.R. *Biochemistry* **1977,** *16*, 1642-1648
4. Haynie, S.L.; Crum, G.A.; Doele, B.A. *Antimicrobial Agents and Chemotherapy 1995, 39, 301-307*
5. Appendini, P.; Hotchkiss, J.H. *Journal of Applied Polymer Science* **2001,** *81,* 609-616.
6. Aymonier, C.; Schlotterbeck, U.; Antonietti, L.; Zacharias, P.; Thomann, R.; Tiller, J.C.; Mecking, S. *Chemical Communications* **2002,** 3018-3019.
7. Zasloff, M. *Nature* **2002,** *415*, 389-395.
8. Fernandez-Lopez, S.; Kim, H.S.; Choi, E.C.; Delgado, M.; Granja, J.R.; Khasanov, A.; Kraehenbuehl, K.; Long, G.; Weinberger, D.A.; Wilcoxen, K.M.; Ghadiri, M.R. *Nature* **2001,** *414,* 329-329.
9. Hamuro, Y.; Schneider, J.P.; DeGrado, W.F. *Journal of the American Chemical Society* **1999,** *121,* 12200-12201.
10. Raguse, T.L.; Porter, E.A.; Weisblum, B.; Gellman, S.H. *Journal of the American Chemical Society* **2002,** *1241,* 12774-12785.
11. Epand, R.F.; Umezawa, N.; Porter, E.A.; Gellman, S.H.; Epand, R.M. *European Journal of Biochemistry* **2003,** *270,* 1240-1248.
12. Patch, J.A.; Barron, A.E. *Journal of the American Chemical Society* **2003,** *125*, 12092-12093.
13. Woo, G.L.Y.; Mittelman, M.W.; Santerre, J.P. *Biomaterials* **2000,** *21,* 1235-1246.
14. Tew, G.N.; Liu, D.H.; Chen, B.; Doerksen, R.J.; Kaplan, J.; Carroll, P.J.; Klein, M.L.; DeGrado, W.F. *Proceedings of the National Academy of Sciences of the United States of America* **2002,** *99,* 5110-5114.
15. Tiller, J.C.; Liao, C.J.; Lewis, K.; Klibanov, A.M. *Proceedings of the National Academy of Sciences of the United States of America* **2001,** *98,* 5981-5985.
16. Lin, J.; Qiu, S.Y.; Lewis, K.; Klibanov, A.M. *Biotechnology Progress* **2002,** *18*, 1082-1086.
17. Lin, J.; Qiu, S.Y.; Lewis, K.; Klibanov, A.M. *Biotechnology and Bioengineering* **2003,** *83*, 168-172.
18. Lin, J.; Murthy, S.K.; Olsen, B.D.; Gleason, K.K.; Klibanov, A.M. *Biotechnology Letters* **2003***, 25,* 1661-1665.
19. Gottenbos, B.; Grijpma, D.W.; van der Mei, H.C.; Feijen, J.; Busscher, H.J. *Journal of Antimicrobial Chemotherapy* **2001**, *48,* 7-13*.*
20. Obrien-Simpson, N.M.; Ede, N.J.; Brown, L.E.; Swan, J.; Jackson, D.C. *Journal of the American Chemical Society* **1997**, *119*, 1183-1188.
21a) Ayres, L.; Vos, M.R.J.; Adams, P.J.H.M.; Shklyarevskiy, I.O.; van Hest, J.C.M. *Macromolecules* **2003**, *36*, 5967-5973. b) Ayres, L. ; Adams, P J. H; M.; Löwik, D.W.P.M. ; van Hest, J.C.M: "β-Sheet Side Chain Polymers Synthesized by ATRP" *Biomacromolecules,* in print. c) Ayres, L.; Koch, K.; Adams, P.J.H.M.; van Hest, J.C.M: "Stimulus Responsive Behaviour of Elastin Based Side Chain Polymers" *Macromolecules,* in print.
22. Raja, A. ; LaBonte, J.; Lebbos, J.; Kirkpatrick, P. *Nature Rev. Drug. Discov.* **2003**, *2*, 943-944
23a) Grotenbreg, G.M.; Spalburg, E.; de Neeling, A.J.; van der Marel, G.A.; Overkleeft, H.S.; van Boom, J.H.; Overhand, M. *Bioorganic & Medicinal Chemistry* **2003**, *11*, 2835-2841.b) Grotenbreg, GM.; Christina; A.E., Buizert; A.E.M., van der Marel , G.A.; Overkleeft , H.S.;
   Overhand M. "Synthesis and Application of Carbohydrate Derived Morpholine Amino Acids", in print
24a) Grotenbreg, G.M.; Timmer, M.S.M.; Llamas-Saiz, A.L.; Verdoes, M.; van der Marel, G.A.; van Raaij, M.J.; Overkleeft, H.S.; Overhand, M. *Journal of the American Chemical Society* **2004**, *126,* 3444-3446.b) Grotenbreg, G.M.; Kronemeijer, M.; Timmer, M.S.M.; El Oualid, F.; van Well, R.M.; Verdoes, M.; Spalburg, E.; van Hooft, P.A.V.; de Neeling, A.J.; Noort, D. ; van Boom, J.H. ; van der Marel, G.A.; Overkleeft, H.S.; Overhand, M. "TA Practical Synthesis of Gramicidin S and Sugar Amino Acid Containing Analogues". In print.
25. Porter, E.A.; Weissblum, B.; Gellman S.H. *J. Am. Chem. Soc.* **2002,** *124,* 7324-7330.
26. Schmitt, MA; Weissblum B.; Gellman S.H. *J. Am. Chem. Soc.* **2004**, *126,* 6848-6849.
27. Seebach, D,.; Beck ,A.K.; Bierbaum, D.J. *Chem&Biodivs* **2004,** *1,* 1111.
28. Aguilera, B.; Wolf, L.B.; Nieczypor, P.; Rutjes, F.P.J.T.; Overkleeft, H.S.; van Hest, J.C.M.; Schoemaker, H.E.; Wang, B.; Mol, J.C.; Furstner, A.; Overhand, M. ; van der Marel, G.A.; van Boom J.H. J. *Org. Chem.* **2001**, *66*, 3584
29. Aguilera, B.; Siegal, G.; Overkleeft, H.S.; Meeuwenoord, N.J.; Rutjes, F.P.J.T.; van Hest, J.C.M.; Schoemaker, H.E.; van der Marel, G.A.; van Boom J.H. Overhand, M. *Eur. J. Org. Chem.* **2001,** *8,* 1541
30. El Oualid F.; Bruining L.; Leroy I.M.; Cohen L.H.; van Boom J.H.; van der Marel G.A.; Overkleeft H.S.; Overhand M. *Helv. Chim. Acta* **2002,** *85,* 3455
31. van Well R.M.; Overkleeft H.S.; van der Marel G.A.; Bruss D.; Thibault G.; de Groot P.G.; van Boom J.H.; Overhand M. *Bioorg. Med. Chem. L*e*tt.* **2003,** *13*, 331.
32. van Well R.M.; Marinelli L.; Altona C.; Erkelens K.; Siegal G.; van Raaij M.; Llamas-Saiz A.L.; Kessler H.; Novellino E.; Lavecchia A.; van Boom J.H.; Overhand M. *J. Am. Chem. Soc.* **2003,** *125*, 10822.
33. Turner J.J.; Sikkema F.D.; Erkelens K.; van der Marel G.A.; Overkleeft H.S.; van Boom J.H.; Overhand M. *Chem. Lett.* **2004**, *33*, 468.
34. El Oualid F.; Burm B.E.A.; Leroy I.M.; Cohen L.H.; van Boom J.H.; van den Elst H.; Overkleeft H.S.; van der Marel G.A.; Overhand M. *J. Med. Chem.* **2004,** *47*, 3920.
35a) Zhang, L. ; Rozek, A.; Hancock, R.E.W. *J. Biol. Chem.* **2001,** *276,* 35722-35722. b) Jung, D.; Rozek, A.; Okon, M.; Hancock, R.E.W. Chem&Biol **2004,** *11,* 949-957.
36. Gottenbos, B.; van der Mei, H. C.; Klatter, F.; Grijpma, D.W.; Feijen, J.; Nieuwenhuis, P.; Busscher, H.J. *Biomaterials* **2003,** *24,* 2707-2710.
37. Neut, D.; van de Belt, H.; van Horn, J.R.; van der Mei, H.C.; Busscher, H. *J. Acta Orthopaedica Scandinavica* **2003,** *74*, 670-676.
38. Roosjen, A.; Kaper, H.J.; van der Mei, H.C.; Norde, W.; Busscher, H.J. *Microbiology-Sgm* **2003,** *149*, 3239-3246.
39. Coessens, V. et al. Prog. Polym. Sci. 2001, Vol 26, pp: 337-377.
40. Craik DJ *et. al.* 2003; J. of Bacteriol Vol 184(14) pp:4011-4021
41. Li P. *et. al.* (2002) Current Organic Chemistry, April 2002, vol. 6, no. 5, pp. 411-440(30): Bentham Science Publishers.
42. Sanda F, Endo T; Macromol.Chem. Phys. 200, 2651-2661 (1999)
43. Hopkins TE and Wagner KB Macromolecules (2003) 36, 2206-2214
44. Gao G. et al Macromolecules (2003) 36, 3938-3943.
45. Patch and Barron (2002) Mimicry of bioactive peptides via non-peptidomimetic oligomers; Current Opinion in Chemical Biology, Vol 6:872-877.
46. Matsoukas *et al* (2001) Designing Peptide Mimetics for the Treatment of Multiple Sclerosis; Mini Reviews in Medicinal Chemistry, **2001,** *1*, 273-282. Bentham Science Publishers Ltd
47. Adv. Mat. **1997,** *9*, 299,
48. Rutjes F.P.J.T., Wolf L.B., Schoemaker H.E. JOURNAL OF THE CHEMICAL SOCIETY-PERKIN TRANSACTIONS 1 (24): 4197-4212 2000.
49. Wang L, Schultz P.G. ANGEWANDTE CHEMIE-INTERNATIONAL EDITION 44 (1): 34-66 2005.
50. James S Chimica Oggi (2003), 21(6), 65-68.
51. Ma, Hongwei; Hyun, Jinho; Stiller, Philip; Chilkoti, Ashutosh. Advanced Materials (2004), 16(4), 338-341. **Potential coatings applications for polymers prepared by ATRP.**
52. Coca, Simion; Woodworth, Brian E. PPG Industries, Inc. USA, Polymeric Materials Science and Engineering (2004), 90 182.

## Claims

1. A method for producing a polymer comprising polymerising at least two monomers to an initiator by means of radical polymerisation in the presence of a capping agent that is reversibly associated with the growing polymer chain, wherein at least one of said monomers comprises a (poly)peptide or a functional equivalent thereof comprising at least 7 amino acids or functional equivalents thereof.

2. A method according to claim 1, wherein said polymerisation step is performed in the presence of a metal complex that reversibly associates with said capping agent thereby providing said method with a switching mechanism that switches said polymer between a not growing capped (dormant) state and an uncapped growing (living) state.

3. A method according to claim 1 or claim 2, wherein said polymerisation is done by means of atom transfer radical polymerisation (ATRP).

4. A method according to any one of claims 1-3, wherein said peptide comprises at least 10 amino acids or functional equivalents thereof.

5. A method according to claim 4, wherein said peptide is a cyclopeptide.

6. A method according to any one of claims 1-5, wherein said functional equivalent of a peptide comprises a peptidomimeticum.

7. A method according to any one of claims 1-6, wherein said functional equivalent of an amino acid comprises a β-amino acid.

8. A method according to any one of claims 2-7, wherein said metal complex comprises a multiamine ligand, preferably HMTETA or PMDETA

9. A method according to any one of claims 2-8, wherein said polymerisation is performed at a temperature of at least 40 °C.

10. A polymer comprising at least one peptide or a functional equivalent thereof comprising at least 7 amino acids or functional equivalents thereof in a side chain.

11. A polymer according to claim 10, obtainable by a method according to any one of claims 1-9.

12. A polymer according to claim 10 or claim 11, wherein said peptide comprises bacteriostatic activity.

13. A polymer according to claim 12, wherein said peptide comprises bactericidal activity.

14. Use of a polymer according to any one of claims 10-13, for interacting with biological material.

15. Use of a polymer according to any one of claims 10-13, for coating a surface.

16. Use of a polymer according to any one of claims 10-13, for at least inhibiting bacterial growth.

17. A method for at least inhibiting bacterial growth on a surface comprising providing said surface with a polymer according to any one of claims 10-13.

18. A method according to claim 17, wherein said surface is in contact with an individual.

19. An apparatus for contacting an individual comprising a polymer according to any one of claims 10-13 for at least inhibiting bacterial growth.

20. An apparatus according to claim 19, wherein said apparatus comprises tubing coated with a polymer according to any one of claims 10-13.

21. A device comprising a surface for use at an interface between a bacteria free and a bacteria containing environment, said surface comprising a polymer according to any one of claims 10-13.

22. A medical device according to claim 21.

23. A device according to claim 21 or claim 22 that is disposable.

24. A coating comprising a polymer according to any one of claims 10-13.

25. Use of a polymer according to any one of claims 9-13 as a protective layer for flower bulbs, as a textile coating, as antibiotic bone cement and/or as antibiotic coating for a medical device.

26. A monomer that is polymerisable through radical polymerisation comprising in a side chain of said monomer at least 7 amino acids in peptidic linkage with each other.
